# EUROPEAN PATENT APPLICATION

(11) **EP 4 763 247 A1**
(43) Date of publication of application: **24.06.2026**
(21) Application number: 25224915.6
(22) Date of filing: 18.12.2025
(51) Int. Cl.: A61M 5/31

(54) **SYRINGE FOR THE MEDICAL FIELD**

(30) Priority: 20.12.2024 IT 202400029370
(71) Applicant: STEVANATO GROUP S.P.A., 35017 Piombino Dese (PD) (IT)
(72) Inventor: BENIGNI, Michele, 30030 Salzano (VE) (IT); CELEGHIN, Linda, 35018 San Martino Di Lupari (PD) (IT)
(74) Representative: Riccardi, Elisa

(57) **Abstract**

A syringe (10) comprising a body (12) defining a liquid containing chamber (14) and a tip (16) protruding from the body (12) and configured to fluid-tightly couple with a female connector of or for a medical device, wherein a through channel (18) is formed through the tip (16) and is in fluid communication with the chamber (14) at a chamber aperture (20) and with the external environment at an end aperture (22). The through channel (18) is formed of a frusto-conical portion (60) extending from the chamber aperture (20) to a longitudinally intervening section (62), and of a cylindrical portion (64) extending from the longitudinally intervening section (62) to the end aperture (22), the frusto-conical portion (62) being tapered toward the chamber aperture (20).

## Description

The invention relates to the field of syringes for the medical field and of manufacture thereof, in particular of liquid dispensing syringes.

A syringe comprises in general an elongate body defining a liquid containment chamber, wherein a plunger is usually sealingly slidable, and a tip, also named syringe conus or fitting, protruding from a longitudinal end of the body and provided with a through channel in fluid communication with the chamber. The tip is configured to fluid-tightly coupling with a female connector of a medical device such as a catheter, a phleboclysis set, or other medical device for dispensing a liquid to a patient, or alternatively with a female connector of a device for drawing a liquid from a patient, such as for example a needle. The connector may be an integral part of the medical device or it may be a stand-alone piece.

Syringes having a Luer type tip are very widespread, providing for a tapered frusto-conical surface having a 6% taper, which couples into a frusto-conical bore of a female connector.

In the present description and the attached claims, under "taper", expressed as a percentage, the change in diameter in a given unit of measurement along a length equal to 100 in the unit of measurement is meant.

In the case of luer slip tips, coupling is by interference fit.

In the case of luer lock tips, a threaded coupling radially outer with respect to the conical coupling is further provided, serving to ensure firmness of the coupling of the syringe with the medical device or its connector, preventing unintentional detachment thereof in axial direction. The threaded coupling occurs between an outer thread of the female connector and an inner thread made in a collar which is formed as one piece with the syringe (typically in the case of plastic syringes) or in an adapter suitably bound to the tip (typically in the case of glass syringes), for example accommodated in a circumferential groove at the basis of the tapered frusto-conical surface.

Said tips and said connectors are described in ISO (International Organization for Standardization) standard 80369-7:2021.

In some cases, the female connector is provided with a cannula (sometimes called "pin") protruding within the bore of the connector itself, which cannula is to be inserted, at least partially, into the through channel of the syringe tip. This particular configuration offers some advantages, such as greater stability and fluid-tightness, ease of administering the liquid dispensed by the syringe, containment of infections.

The connectors provided with a cannula protruding within the bore are usually used for intravascular administrations and/or in situations requiring a particularly secure and reliable connection, such as for example administration of drugs under high pressure and/or of high viscosity liquids, or in procedures where several movements are required. In some cases, the cannula is protected by a retractable sheath, which is pushed away by the coupling with the syringe tip.

However, the through channel and the cannula are not subject to standardization, and their sizes are conversely left to any agreement between the syringe manufacturers and the manufacturers of medical devices or of connectors for the latter, with the consequence of rendering the coupling of a given syringe with connectors different from the specific ones for which it has been manufactured inadequate, if not even impossible.

Document US 11,660,402 B2 discloses a syringe having a cylindrical portion protruding from a body portion and having a tapered inner diameter and including a female luer section. In an embodiment, at the end of the cylindrical portion on the side of the body portion, there is an annular section protruding inward to radially compress a sealing section of a syringe cap, made of an elastic material. In another embodiment, the radially protruding section is replaced by a recess.

Document GB2495492A discloses a syringe, in particular for enteral administrations, with a non-luer type tip, having a tapered bore with a taper different from 6%.

Document US 5,624,402 disclosed a syringe tip cap, the syringe having means for coupling with the cap or a needle, including a luer collar that is unitarily formed with the syringe or that is securely mounted to the syringe in proximity to the tip.

EP 2 320 992 B1**,** which is considered the closest prior art with respect to the invention, discloses a syringe defining a reservoir for containing a liquid and comprising an end-piece having a longitudinal axis A, the said end-piece encompassing a channel aligned with the said longitudinal axis A and providing a passageway for the transfer of the said liquid, the said channel comprises at least two portions, a first portion and a second portion, the said first portion extending from a free distal end of the end-piece in the direction of the reservoir, and the said second portion extending between the first portion of the channel and a proximal end of the end-piece and connecting the said first portion to the reservoir, the said first portion having an average diameter that is greater than the average diameter of the said second portion, each of said first and second portions having the shape of a cylinder so that, for each portion, the average diameter is substantially constant, wherein a transition zone connects the said first portion and the said second portion together, the said transition zone having a length along the longitudinal axis A of the said end-piece greater than 0, wherein the diameter of the said transition zone varies along the said length within a range defined by the average diameter of the said first portion and the average diameter of the said second portion, and wherein the syringe and the end-piece are made in a single piece out of glass. According to the document, such cylindrical portions allow a better flow of the medical liquid when expelled through the passageway, an easy manufacturing, a suitable connection with connectors comprising an internal canula that have the shape of a cylinder and a reduction of the dead volume. According to the document, the first portion of the channel allows compatibility with a connector and the second portion confers on the end-piece sufficient strength to prevent breakage when it is inserted into a conical assembly, for example in a female 6% Luer conical assembly.

The Applicant notes that the first portion is subject to the above highlighted drawbacks of poor compatibility with several connectors, for example of different manufacturers. Furthermore, the Applicant notes that the liquid, entering the transition zone after having passed through the second portion, is subject to a sudden deceleration and to a remarkable pressure increase due to the sudden widening to the larger cross-section corresponding to that of the first portion; furthermore, due to the inertia and the viscous friction, the peripheral liquid particles, closer to the channel walls, loose speed faster than the paraxial ones, and tend to detach from the walls. Retrograde or stagnant flow zones ensue, in which vortices form and, as a consequence, the liquid suction by the cannula at the transition zone is disadvantageously subject to turbulence.

The technical problem at the basis of the invention is to overcome the drawbacks of the prior art, making a syringe available which has a tip capable of ensuring an adequate connection with a wide range of connectors of or for medical devices, having cannulae of different geometries and/or sizes protruding within the respective bore for coupling with the syringe tip - besides possibly with connectors free of cannula.

A secondary objective of the invention is to favour the dispensing of a liquid from the syringe.

Another secondary objective of the invention is to allow the through channel to be made with a tool having a simple shape.

The invention relates to a syringe comprising a body, for example an elongate body, defining a liquid containment chamber and a tip protruding from the body, for example from a longitudinal end of the body, and configured to fluid-tightly couple with a female connector of or for a medical device, wherein a through channel is formed through the tip, for example extends about and along the longitudinal axis of the tip, and is in fluid communication with the chamber at a chamber aperture and with the external environment at an end aperture. The through channel is formed of a frusto-conical portion extending from the chamber aperture to a longitudinally intervening section, and of a cylindrical portion extending from the longitudinally intervening section to the end aperture, the frusto-conical portion being tapered toward the chamber aperture.

The cylindrical portion turns out to be adjacent and contiguous to the frusto-conical portion.

In the following description and claims, term "comprising" also includes terms "essentially consisting of" or "consisting of".

In the present description and in the attached claims, expression "to be intervening" indicates that the subject is included in the space extending from one complement to the other one, but is neither meant in a limiting sense, nor to indicate a position equidistant from the complements, nor to indicate that in such space there is only the subject. Such an expression should therefore not be meant as implying equidistant from two extremes. In the specific case, the longitudinally intervening section is not necessarily equidistant from the chamber aperture and the end aperture.

In use, the cannula of the female connector is accommodated in the cylindrical portion, more external, and may possibly insert to a certain extent in the frusto-conical portion, depending on the maximum cross size of the cannula. The cannula of the female connector may be pushed until it interference fits with the syringe tip at the frusto-conical portion, so as to increase the coupling firmness also in case of a particularly small cannula with respect to the cross sizes of the channel. On the other hand, in the case of a larger cannula, it may interference fit with the tip already at the cylindrical portion, abutting on the frusto-conical portion at the intervening section.

Said geometry of the through channel therefore allows to easily adapt to female connectors - both of the luer type and of non-luer type - having cannulae, coaxial with and protruding within the respective bore, having different shapes and/or different sizes, for example, having cylindrical cannulae of different length and/or diameter, frusto-conical cannulae of different length, average diameter and/or slant of the external surface, cannulae having an outer contour more or less complex.

Furthermore, thanks to the fact that the frusto-conical portion may be made with a sufficiently small half-angle, since no further cylindrical section of the through channel on the chamber side is required, the liquid flow may be substantially homogeneous as far as speed and pressure are concerned, not turbulent, across the entire cross-section, and be affected to a negligible extent by the slowdown at the channel walls, thus without forming vortexes or in any case with a very limited formation.

In the present description and the attached claims, under "half-angle" of the frusto-conical portion, the slant of the wall with respect to the longitudinal axis of the frusto-conical portion in an axial section across the frusto-conical portion is meant.

The geometry of the through channel, being free of sudden variations and of undercut portions, does not entail pressure drops, or in any case minimizes the same, so that, when the medical device comprises a suction or pushing pump of the liquid from the syringe, the pump does not work under stress.

Such a geometry of the through channel may advantageously be obtained through a particularly simple tool, which in case of glass syringes consists of a drilling tip of the glass in the softened state, and in the case of plastic syringes consists of a mould.

Furthermore, the provision of the frusto-conical portion on the side of the body allows to keep enough wall thickness of the syringe tip to avoid undesired breakages while forming the tip, in particular when it is made of glass.

The provision of the frusto-conical portion also allows to keep the dead volume small, namely the volume of liquid that, with a plunger fully inserted in the chamber, remains within the through channel of the tip.

The medical device may be a dispenser medical device, preferably selected among a pump device, a catheter, a phleboclysis set, and possibly an injection needle.

The frusto-conical portion may have a half-angle comprised between 1.5° and 8.5°, preferably between 2.5° and 7.5°, more preferably between 3.3° and 5°, even more preferably equal to 4.14°.

In the following description and claims, the definitions of the numerical ranges comprise the individual values within the range and its extremes, unless otherwise specified.

The length ratio between the cylindrical portion and the frusto-conical portion may be comprised, for example, between 1.00 and 1.60, preferably between 1.30 and 1.50, more preferably it is equal to 1.45.

The chamber may comprise a narrowing flowing into the through channel.

The end aperture may be flared.

A plunger may be slidable in the chamber

The plunger may be fixed to an operating rod protruding from an aperture of the chamber opposed to said chamber aperture.

The plunger, at end of stroke, may enter at least partly in the narrowing of the chamber.

The tip preferably embodies a male connector.

The male connector, and thus the external surface of the tip, may have a frusto-conical length, for example configured to couple into a frusto-conical bore of the female connector.

The frusto-conical length may have 6% taper, preferably the male connector is of the luer type as defined by ISO standard 80369-7:2021.

The male connector may comprise a circumferential groove defining an undercut, preferably the male connector may be of the luer lock type as defined by ISO standard 80369-7:2021. Such a groove may serve to accommodate an adaptor which, in use, is threadedly coupled with a threading of the female connector.

The male connector may comprise, alternatively to the groove, an internally threaded collar, arranged coaxial with and radially outer to the frusto-conical length, preferably the male connector may be of the luer lock type as defined by ISO standard 80369-7:2021. In use, such a threaded collar is threadedly coupled with a threading of the female connector.

Both technical solutions mentioned above ensure firmness of the coupling of the syringe with the female connector of or for the medical device.

The tip may be formed as one piece with the body of the syringe.

The syringe may be made of glass, for example borosilicate glass.

Alternatively, the syringe may be made of plastics, for example cyclic olefin polymer (COP), cyclic olefin copolymer (COC), polycarbonate (PC), polypropylene (PP), polyethylene (PE) and combinations thereof.

The chamber may be cylindrical and the oblong body may be provided with symmetry with respect to a longitudinal axis.

Features and advantages of the present invention will become clearer from the following detailed description of an embodiment thereof, made with reference to the attached drawings, wherein:
- fig. 1 schematically shows a syringe and a medical device connected to the syringe for treating a patient,
- fig. 2 is a cross-sectional view of a syringe tip,
- fig. 3 is a cross-sectional view of the syringe tip of fig. 2, with connected connector.

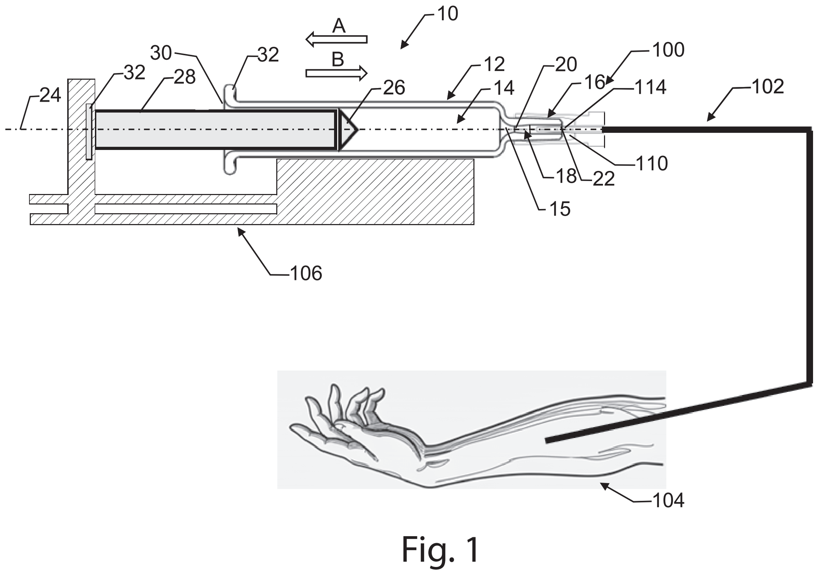

With reference to **fig. 1****,** a syringe 10 comprises a body 12, for example oblong, defining a liquid containment chamber 14 and a tip 16 protruding from the body 12, for example from a longitudinal end thereof, where the chamber 14 has, in the case shown, a narrowing 15.

The tip 16 is, for example, made as one piece with the body 12.

The syringe 10 and the tip 16 may be made of glass, for example borosilicate glass, or of plastics, for example cyclic olefin polymer (COP), cyclic olefin copolymer (COC), polycarbonate (PC), polypropylene (PP), polyethylene (PE) and combinations thereof.

A through channel 18 is formed through the tip 16 and is in fluid communication with the chamber 14 at a chamber aperture 20 (to the left in fig. 1) and with the external environment at an end aperture 22 (to the right in fig. 1).

Both the tip 16 with the through channel 18 and the chamber 14 typically extend about and along a longitudinal axis 24 of the syringe 10, as shown. Both the through channel 18 and the chamber 14 typically have the shape of rotation solids about the longitudinal axis 24, as shown, and are therefore provided with symmetry with respect to the longitudinal axis 24. However, it is possible to provide for different shapes, for example the chamber 14 may have an oval section and/or the tip 16 and/or the through channel 18 may be offset with respect to the longitudinal axis 24.

The tip 16 is configured to fluid-tightly couple, in use, with a connector 100 of or for a medical device 102, for treatment of a patient, whose arm 104 is shown.

As far as the medical device 102 is concerned, it may be a dispenser medical device such as a pump device (cf. pump 106), a catheter or a phleboclysis set. However, it may also simply be an injection needle.

In the case shown, a plunger 26 is sealingly slidable in the chamber 14 of the syringe 10. The plunger 26 preferably has a tapered shape so that it can enter at least in part in the narrowing 15 of the chamber 14, also thanks to its deformability.

In the case shown, the plunger 26 can be driven to slide through an operating rod 28 partly protruding from the chamber 14, at an aperture 30 thereof at the opposite end to that where there is the tip 16. Protrusions 32 (or a collar) may protrude radially from the body 12 at such an aperture 30, in order to provide finger abutment surfaces or grip elements for the operator of the syringe 10. The plunger 26 defines a filled region of the chamber 14, extending between the plunger 26 and the tip 16, and an empty region of the chamber 14, extending between the plunger 26 and the opposite end of the chamber 14 (towards the aperture 30), the filled and empty regions having a variable volume according to the position of the plunger 26.

When the syringe 10 is used as a dispenser syringe, application where the invention proves to be of particular advantage, the chamber 14 is initially filled with a liquid dose and, after connection with the medical device 102, the plunger 26 is pushed (or pulled) in direction B of pushing the liquid out of the chamber 14 and out of the syringe 10, through the through channel 18 in the tip 16, so as to administer the liquid to the patient 104.

When the syringe 10 is used as a suction syringe, it is initially empty and, after connection with the medical device 102, the plunger 26 is pulled (or pushed) in direction A of (partly) extraction from the chamber 14, so as to fill the chamber 14 at least partly with a liquid drawn from the patient 104.

In other cases, the plunger 26 may be free of operating rod 28 and be sucked together with the liquid by the medical device 102, for example by the action of a pump thereof.

The tip 16 embodies for example a male connector, while the connector 100 is a female connector, provided with a bore 110 configured to receive the tip 16.

In the case shown, the connector 100 is provided with a cannula 114, also named pin, protruding within bore 110. In use, the cannula 114 is inserted, at least partially (as shown), in the through channel 18 of the tip 16 of the syringe 10, so as to make the connection of the connector 100 and in general of the medical device 102 with the tip 18 and in general with the syringe 10 more firm and sterile. In order to increase sterilization, a retractable sheath (not shown) may be provided for protection of the cannula 114, which is pushed away by the coupling with the tip 16 of the syringe 10.

The syringe 10 may however also be used with a connector 100 free of cannula 114.

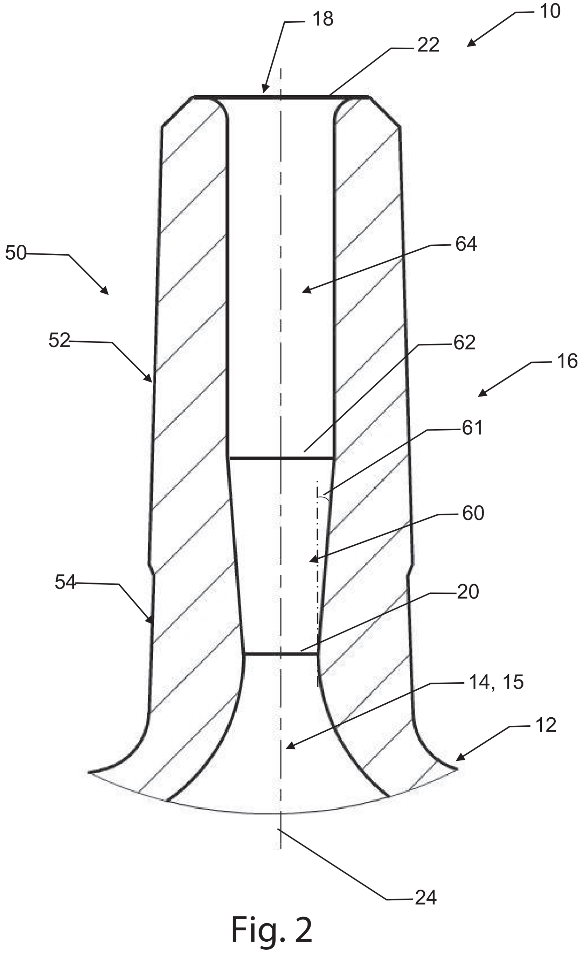

With reference to **fig. 2****,** tip 16 embodies, as mentioned, a male connector 50.

The male connector 50, and therefore the outer surface of the tip 16, has a frusto-conical length 52, preferably having 6% taper, so as to comply with ISO standard 80369-7:2021 referred to above (luer type tip). The frusto-conical length is, for example, a preponderant length 52 of the tip 16 itself.

In a specific, not limiting example, the frusto-conical length 52 has a length equal to about 7.8 mm and diameter variable between about 4.04 mm (measured at a distance of 1.5 mm from the free end of the frusto-conical length 52 itself) and 4.42 mm, with a tolerance for each diameter equal to 0.07 mm.

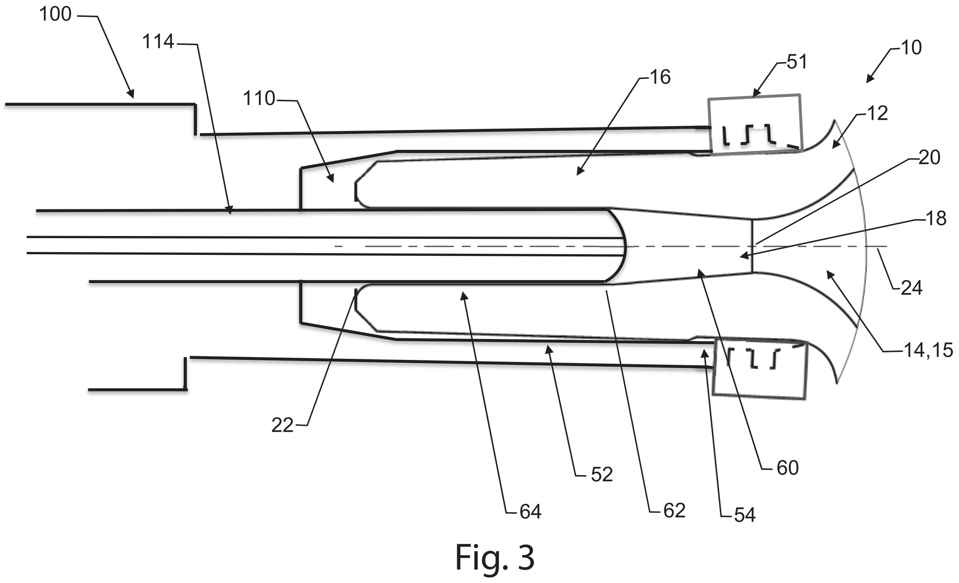

The male connector 50 may comprise a circumferential groove 54. The circumferential groove 54 is for example made, as shown, at the base of the tip 16 (at the base of the frusto-conical length 52), namely on the side of the body 12 of the syringe 10. Such a groove 54, by defining an undercut, may receive an adapter (cf. adapter 51 in fig. 3) having an internally threaded collar, configured to couple with an outer threading of connector 100, so as to increase firmness of the coupling between the two connectors 50, 100, in particular against undesired removal the tip 16 from connector 100. In this way, tip 16 complies with ISO standard 80369-7:2021 referred to above (luer lock type tip).

Also the circumferential groove 54 may have a frusto-conical shape.

Syringe 10 may however be made with a male connector different from a Luer type connector and is therefore in general suitable to be connected both to Luer type connectors and to non-Luer type connectors.

The through channel 18 of the tip 16 is formed of a frusto-conical portion 60 extending from chamber aperture 20 to a longitudinally intervening section 62, and of a cylindrical portion 64 extending from the longitudinally intervening section 62 to the end aperture 22, which is typically slightly flared as shown. The cylindrical portion 64 turns out to be adjacent and contiguous to the frusto-conical portion 60.

The frusto-conical portion 60 is tapered toward the chamber aperture 20.

The frusto-conical portion 60 has a half-angle 61 comprised between 1.5° and 8.5°, preferably between 2.5° and 7.5°, more preferably between 3.3° and 5°, even more preferably equal to 4.14°. As is manifest from Fig. 2, the half-angle 61 represents the slant of the wall of the frusto-conical portion 60 with respect to the longitudinal axis 24 in an axial section across the frusto-conical portion 60.

As already noted, the longitudinally intervening section 62 is not necessarily equidistant from the chamber aperture 20 and the end aperture 22, and preferably it is not.

The length ratio (the lengths being taken along the longitudinal axis 24) between the cylindrical portion 64 and the frusto-conical portion 60 may vary, for example, between 1.00 and 1.60, preferably between 1.30 and 1.50, more preferably is equal to 1.45.

In an example case, however not limiting, the diameter of the through channel 18 at the chamber aperture 20 is equal to 1.25 mm and at the intervening section 62 is equal to 1.8 mm; furthermore, the frusto-conical portion 60 is 3.8 mm long; the frusto-conical portion 60 therefore has a half-angle equal to 4.14°.

In such a case, the cylindrical portion 64 is 5.5 mm long and the above length ratio is equal to about 1.45.

The chamber 14 may have, for example, a volume comprised between 0.5 ml and 20 ml, preferably between 1 and 5 ml.

It is worthwhile emphasizing that sizing the through channel 18 and its portions is independent of sizing the chamber 14.

**Fig. 3** shows a cross-sectional view of the tip 16 of the syringe 10, in the state coupled with a female connector 100 of or for a medical device 102.

An adapter 51 is also schematically shown, having a collar threadedly coupled with the female connector 100 and axially retained in the groove 54 of the tip 16, so that the female connector 100 is retained against removal in axial direction 24 thanks to the undercut.

In the case shown, the female connector 100 is of the luer lock type, complying with ISO standard 80369-7:2021, having a conical bore 110, with taper equal to 6%.

In the case shown, adapter 51 is a Luer adapter, complying with ISO standard 80369-7:2021. The adapter 51 may have an axial size (length) equal to or less than that of the circumferential groove 54, so as to allow a certain freedom in the coupling depth of the female connector 100 with the tip 16 along axis 24. The coupling depth may moreover be varied to a certain extent by varying the screwing depth of the female connector 100 into the adapter 51.

In another cases, for example when the syringe 10 is made of a plastic material, adapter 51 may be replaced by an internally threaded collar made as one piece with the rest of the syringe 10 itself, coaxial with and radially outer of the tip 16.

In the case shown, the female connector 100 comprises a cannula 114 protruding within the conical bore 110.

In the coupled condition shown in Fig. 3, the cannula 114 is partially inserted in the through channel 18 of the tip 16 of the syringe 10. Specifically, the cannula 114 extends through the entire cylindrical portion 64 of the through channel 18 and abuts on the frusto-conical portion 60 at the intervening section 62. The cannula 114 has a diameter substantially equal to that of the cylindrical portion 64, just smaller so as to allow its insertion.

It is understood that in case the cannula 114 had a smaller diameter than that shown, it would also extend in a length of the frusto-conical portion 60, until coupling by interference fit with the inner wall thereof.

The specific geometry of the through channel 18 of the tip 16 therefore allows to easily adapt to female connectors 100 having cannulae 114, coaxial with and protruding within the respective bore 110, having different shapes and/or different sizes, besides also allowing coupling with connectors 100 free of cannula.

Considering a dispenser syringe, the suction point of the liquid into the cannula 114 and thus into the medical device 102 lies in the frusto-conical portion 60, at or in proximity of the intervening section 62. Given that the frusto-conical portion 60 already starts at the chamber 14, the length at disposal in the tip 16 is such as to allow it to be made with a half-angle 61 sufficiently small, which favours establishment of a substantially homogeneous liquid flow, as far as speed and pressure are concerned, not turbulent, across the entire cross-section of the through channel 18. The gradual widening of the through channel 18 between the chamber 14 and the suction point by the cannula 114 prevents a remarkable liquid slowdown, especially in the peripheral zones of the through channel 18, and prevents the liquid from detaching from the inner wall of the through channel 18. Therefore, the liquid flow is not subject to formation of vortexes and turbulences. The absence of sudden cross-section changes and of undercut portions along the through channel 18 minimizes, if not even nullifies, the pressure losses, allowing use of the syringe 10 with a medical device comprising a suction or pushing pump of the liquid from the syringe, without the pump having to work under stress.

A homogeneous and non-turbulent liquid flow is however advantageous also in the case of a suction syringe.

As already emphasized in the introductory portion, the through channel 18 formed of the cylindrical portion 64 and the frusto-conical portion 60 may be obtained through a perforation tip of the glass in the softened state having a matching geometry, or by a mould having a matching shape in the case of plastic syringes. Those shapes are advantageously simple.

Because in the proximity of the body 12 of the syringe 10, where the circumferential groove 54 in the tip 16 is provided, the through channel 18 is formed of the frusto-conical portion 60, the wall thickness of the tip 16 is still enough to avoid undesired breakages while forming the tip 16.

Also the dead volume, namely the volume of liquid which, with the plunger 26 fully inserted in the chamber 14 (possibly also within its narrowing 15), remains within the through channel 18 of the tip 16, is minimized thanks to the smaller cross-section of the through channel 18 itself in the frusto-conical portion 60 and to the capability of inserting the cannula 114 of the connector 100 as far as possible into the through channel 18 itself.

Those skilled in the art will understand that, alternatively to the circumferential groove 54, the male connector 50 may comprise an internally threaded collar, arranged coaxial with and radially outer to the tip 16 of the syringe 10, configured to couple with an external threading of the female connector 100 of or for the medical device. Also in this case, firmness of coupling between the two pieces is ensured, in particular against undesired removal of the tip 16 from female connector 100. The collar may be made as one piece with the syringe, especially when it is made of a plastic material. When the male connector 50 has 6% taper, also a syringe provided with such a collar complies with ISO standard 80369-7:2021 referred to above (luer lock type tip).

The male connector 50 may however be free from both the circumferential groove 54 and such a one-piece collar, and couple merely by interference fit with the female connector 100 of or for the medical device. When the male connector 50 has 6% taper, the syringe then complies with ISO standard 80369-7:2021 referred to above (luer slip type tip).

Although aspects of the invention have been described in terms of several specific embodiments, it will be manifest for those skilled in the art that several changes, variations and omissions are possible, without departing from the scope defined by the claims.

## Claims

1. A syringe (10) comprising a body (12) defining a liquid containment chamber (14) and a tip (16) protruding from the body (12) and configured to fluid-tightly couple with a female connector (100) of or for a medical device (102), wherein a through channel (18) is formed through the tip (16) and is in fluid communication with the chamber (14) at a chamber aperture (20) and with the external environment at an end aperture (22), **characterized in that** the through channel (18) is formed of a frusto-conical portion (60) extending from the chamber aperture (20) to a longitudinally intervening section (62), and of a cylindrical portion (64) extending from the longitudinally intervening section (62) to the end aperture (22), the frusto-conical portion (62) being tapered toward the chamber aperture (20).

2. The syringe (10) according to claim 1, wherein the frusto-conical portion (62) has a half-angle (61) comprised between 1.5° and 8.5°, preferably between 2.5° and 7.5°, more preferably between 3.3° and 5°, even more preferably equal to 4.14°.

3. The syringe (10) according to one of the previous claims, wherein the length ratio between the cylindrical portion (64) and the frusto-conical portion (60) is comprised between 1.00 and 1.60, preferably between 1.30 and 1.50, more preferably is equal to 1.45.

4. The syringe (10) according to one of the previous claims, wherein the chamber (14) comprises a narrowing (15) flowing into the through channel (18).

5. The syringe (10) according to one of the previous claims, wherein the end aperture (22) is flared.

6. The syringe (10) according to one of the previous claims, wherein a plunger (26) is slidable in the chamber (14), preferably wherein the plunger (26) is fixed to an operating rod (28) protruding from an aperture (30) of the chamber (14) opposed to said chamber aperture (20) and/or wherein the plunger (26), at end of stroke, may enter at least partly in a or said narrowing (15) of the chamber (14).

7. The syringe (10) according to one of the previous claims, wherein the tip (16) embodies a male connector (50).

8. The syringe (10) according to claim 7, wherein the male connector (50) has a frusto-conical length (52).

9. The syringe (10) according to claim 8, wherein the frusto-conical length (52) has 6% taper, preferably the male connector (50) is of the luer type as defined by ISO standard 80369-7:2021.

10. The syringe (10) according to one of claims 7-9, wherein the male connector (50) comprises a circumferential groove (54) defining an undercut, or wherein the male connector (50) has an internally threaded collar, arranged coaxial with and radially outer to the frusto-conical length (52),
preferably wherein the male connector is of the luer lock type as defined by ISO standard 80369-7:2021.

11. The syringe (10) according to one of the previous claims, wherein the tip (16) is formed as one piece with the body (12).

12. The syringe (10) according to one of the previous claims, made of a material selected among glass, for example borosilicate glass, and plastics, for example cyclic olefin polymer (COP), cyclic olefin copolymer (COC), polycarbonate (PC), polypropylene (PP), polyethylene (PE) and combinations thereof.
